# EUROPEAN PATENT APPLICATION

(11) **EP 3 168 198 A1**
(43) Date of publication of application: **17.05.2017**
(21) Application number: 15867712.0
(22) Date of filing: 05.11.2015
(51) Int. Cl.: C02F 11/12, C02F 3/00, C02F 3/12, C12N 1/00, C12N 1/04

(54) **PROCESS FOR PRODUCING FREEZE-DRIED SLUDGE**

(30) Priority: 11.12.2014 JP 2014250895
(71) Applicant: Mitsubishi Heavy Industries, Ltd., Tokyo 108-8215 (JP)
(72) Inventor: KINUGASA Atsushi, Tokyo 108-8215 (JP); NAKATSUCHI Yuta, Tokyo 108-8215 (JP); KATO Sho, Tokyo 108-8215 (JP); NAKAJIMA Yuuji, Tokyo 108-8215 (JP); OGAWA Naoki, Tokyo 108-8215 (JP); WADA Kyoko, Tokyo 108-8215 (JP)
(74) Representative: Intès, Didier Gérard André
(86) International application number: PCT/JP2015/081165
(87) International publication number: WO 2016/092990

(57) **Abstract**

A process (S1) for producing freeze-dried sludge includes: an acclimatization step (S10) of imposing a load on cells of a microorganism in activated sludge to acclimatize the cells; a production increase step (S21) of subjecting the activated sludge to a production increase operation in which production of an extracellular polymer by the cells is increased after the acclimatization step (S10); and a freeze-drying step (S30) of freeze-drying the activated sludge after the production increase step (S21).

## Description

### [Technical Field]

The invention relates to a process for producing freeze-dried sludge.

Priority is claimed on Japanese Patent Application No. 2014-250895, filed December 11, 2014, the content of which is incorporated herein by reference.

### [Background Art]

Wastewater including organic components discharged from factories, various facilities, equipment, and the like is treated by decomposing the organic components using microorganisms, or the like. Examples of such a wastewater treatment method include a method for decomposing organic components in wastewater and purifying it using activated sludge serving as aggregates of bacteria and the like used to decompose the organic components.

As a wastewater treatment method using activated sludge, for example, Patent Literature 1 discloses a method for decomposing organic components in washing effluent serving as wastewater using a floating activated sludge floc in a biological treatment tank.

### [Citation List]

### [Patent Literature]

[Patent Literature 1]
Japanese Patent No. 3913015

### [Summary of Invention]

### [Technical Problem]

However, when wastewater is treated using activated sludge, treatment performance (decomposing ability) of the activated sludge with respect to organic components included in wastewater needs to be increased. To be specific, the activated sludge is subject to an operation, in which a load is imposed on the activated sludge by continuously providing a treatment target such as organic components included in wastewater to the activated sludge in which bacteria having activity of decomposing components to be treated in the activated sludge have been proliferated in advance. Such a task in which treatment performance of activated sludge is improved refers to acclimatization. Such an acclimatization operation is performed to improve performance of activated sludge when a wastewater treating device using activated sludge starts up or performance of a wastewater treating device in operation deteriorates.

It takes a long period of time until bacteria having activity of decomposing components to be treated have been proliferated in an acclimatization operation. For this reason, in order to shorten an operation time, a method in which activated sludge is subject to an acclimatization operation in advance and the activated sludge having high treatment performance is freeze-dried and used has been studied. To be specific, shortening of an operation time of the acclimatization operation is attempted by adding the freeze-dried activated sludge to water or the activated sludge, which has not been subject to the acclimatization operation.

However, when the activated sludge is freeze-dried, a protective agent such as skim milk or sodium glutamate needs to be externally added to the activated sludge so that a survival rate of bacteria in the activated sludge is not reduced due to deactivation or the like of the bacteria when the bacteria is freeze-dried. As for the protective agent, an appropriate protective medicine needs to be selected in accordance with a type and usage environment of bacteria in the activated sludge. For this reason, a task and costs for adding the protective medicine from the outside are burdensome.

The present invention provides a process for producing freeze-dried sludge in which freeze-dried activated sludge can be obtained without adding a protective medicine from the outside.

### [Solution to Problem]

In order to solve the above-described problems, the present invention suggests the following means.

A process for producing freeze-dried sludge related to a first aspect of the present invention includes: an acclimatization step of imposing a load on cells of a microorganism in activated sludge to acclimatize the cells; a production increase step of subjecting the activated sludge to a production increase operation in which production of an extracellular polymer by the cells is increased after the acclimatization step; and a freeze-drying step of freeze-drying the activated sludge after the production increase step.

With such a constitution, a high load is applied to cells of a microorganism in the activated sludge so that an amount of extracellular polymer in the activated sludge can be increased. An extracellular polymer produced by the cells contains components having a hydrogen bonding group such as a saccharide, a protein, a nucleic acid, and a lipid. For this reason, hydrophilic groups of cytoplasmic bilayer surfaces of the cells are hydrogen-bonded to hydrogen bonding group of the extracellular polymer, the cells are dehydrated, and thus the cells can be reinforced when the activated sludge is freeze-dried. Therefore, the amount of the extracellular polymer in the activated sludge is increased so that many cells in the activated sludge are reinforced, and thus destruction of cell walls of the cells due to moisture at a time of freeze-drying the activated sludge can be suppressed. In other words, the cells in the activated sludge can utilize an extracellular polymer, which has been retained previously, as a protective substance.

In the process for producing the freeze-dried sludge related to a second aspect of the present invention, in the first aspect, the production increase step may have a high-load step of imposing a higher load than that of the acclimatization step on the activated sludge as the production increase operation.

With such a constitution, a load higher than that of the acclimatization step is applied to the cells in the activated sludge so that a concentration of the extracellular polymer can be drastically increased. Therefore, the concentration of the extracellular polymer can be easily increased.

In the process for producing the freeze-dried sludge related to a third aspect of the present invention, in the first or second aspect, the production increase step may have a medicine addition step of adding a medicine in which the cells are proliferated.

With such a constitution, the cells are proliferated by adding the medicine so that the number of cells in the activated sludge can increase and an amount of the extracellular polymer can be increased.

In the process for producing the freeze-dried sludge related to a fourth aspect of the present invention, in any one of the first to third aspect, the process for producing the freeze-dried sludge further includes: a pre-production increase step of subjecting activated sludge for addition to a production increase operation in which the production of the extracellular polymer by the cells is increased, wherein the production increase step may have an addition step of adding an additive generated from the activated sludge for addition after the pre-production increase step to the activated sludge.

With such a constitution, the amount of extracellular polymer in the activated sludge can be increased from an initial stage of the production increase step. Thus, the time taken to increase the production of the extracellular polymer is increased can be shortened in the production increase step. Therefore, the production of the extracellular polymer can be more efficiently increased.

In the process for producing the freeze-dried sludge related to a fifth aspect of the present invention, in the fourth aspect, in the addition step, at least the activated sludge for addition may be directly added as the additive.

With such a constitution, the activated sludge for addition in which the production of the extracellular polymer has been increased is directly added so that the amount of extracellular polymer in the activated sludge can be easily increased.

In the process for producing the freeze-dried sludge related to a sixth aspect of the present invention, in the fourth or fifth aspect, in the addition step, at least the freeze-dried activated sludge for addition may be added as the additive.

With such a constitution, the activated sludge for addition in which the production of the extracellular polymer has been increased can be freeze-dried and added as the additive. For this reason, the activated sludge in which the amount of extracellular polymer has been increased can be reliably added even if a certain amount of time is taken until the production increase step is performed after the pre-production increase step.

In the process for producing the freeze-dried sludge related to a seventh aspect of the present invention, in any one of the fourth to sixth aspect, in the addition step, at least the extracellular polymer extracted from the activated sludge for addition may be added as the additive.

With such a constitution, only the extracellular polymer is added as the additive so that the amount of extracellular polymer in the activated sludge can be more efficiently increased.

### [Advantageous Effects of Invention]

According to a process for producing freeze-dried sludge of the present invention, freeze-dried activated sludge can be obtained by increasing production of an extracellular polymer without adding a protective medicine from the outside.

### [Brief Description of Drawings]

Fig. 1 is a flowchart for describing a process for producing freeze-dried sludge in a first embodiment of the present.
Fig. 2 is a graph showing results of changes in sugar concentration when a load is applied to activated sludge.
Fig. 3 is a flowchart for describing a process for producing freeze-dried sludge in a second embodiment of the present.
Fig. 4 is a flowchart for describing a process for producing freeze-dried sludge in a third embodiment of the present.
Fig. 5 is a flowchart for describing a process for producing freeze-dried sludge in a fourth embodiment of the present.
Fig. 6 is a flowchart for describing a process for producing freeze-dried sludge in a fifth embodiment of the present.

### [Description of Embodiments]

### «First embodiment»

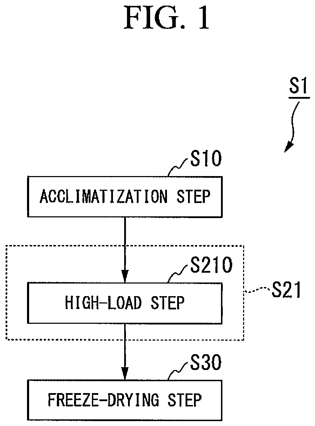

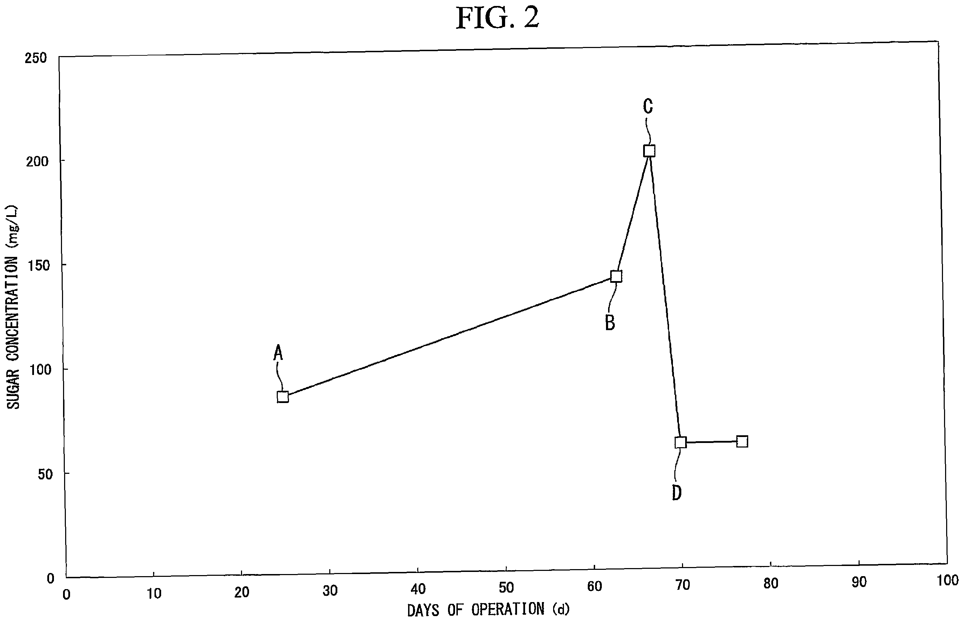

Hereinafter, a first embodiment related to the invention will be described with reference to Figs. 1 and 2.

A process for producing freeze-dried sludge produces freeze-dried sludge (hereinafter referred to as "FD sludge") serving as freeze-dried activated sludge used for purifying wastewater including organic components. As shown in Fig. 1, a process S1 for producing freeze-dried sludge of this embodiment includes an acclimatization step S10, a production increase step S21, and a freeze-drying step S30. In the acclimatization step S10, a load is imposed on cells of a microorganism in activated sludge so that the cells are acclimatized. In the production increase step S21, a production increase operation, in which production of an extracellular polymer by the cells is increased, is performed. In the freeze-drying step S30, the activated sludge is freeze-dried.

In the acclimatization step S10, treatment performance for organic components of the activated sludge is improved. In the acclimatization step S10 of this embodiment, a processing component serving as a processing target such as organic components included in wastewater is applied to the activated sludge so that a load is imposed on the cells. To be specific, in the acclimatization step S10, an amount of organic components greater than a limiting amount for treatment performance for the activated sludge in an initial stage is added to the activated sludge. Subsequently, in the acclimatization step S10, an amount of organic components more than the previous amount of addition is further added to the activated sludge after the added organic components have decomposed. As described above, in the acclimatization step S10, an amount of addition of the organic components added to the activated sludge step by step is increased, and thus a load applied to the cells is gradually increased. Thus, in the acclimatization step S10, the cells are proliferated, and thus the activated sludge with a target treatment performance is acquired.

In the production increase step S21, the activated sludge with the target treatment performance is subjected to the production increase operation in which the production of the extracellular polymer after the acclimatization step S10 is performed. In the production increase step S21, for the production increase operation, environmental conditions are manipulated so that the extracellular polymer is generated using the cells in the activated sludge. The production increase step S21 in this embodiment has a high-load step S210. In the high-load step S210, as the production increase operation, a high load is imposed on the activated sludge after the acclimatization step S10.

In the high-load step S210, a load higher than that of the acclimatization step S10 is applied more rapidly to the cells in the activated sludge. In the high-load step S210 of this embodiment, as the production increase operation, a load which is 1.2 times or more in comparison to the load applied in the acclimatization step S10 is applied to the cells. To be specific, in the high-load step S210, an amount of organic components which is 1.2 or more times the amount added to the activated sludge in the acclimatization step S10 when compared with the limiting amount of the treatment performance to the activated sludge after the acclimatization step S10.

In the freeze-drying step S30, an FD sludge is acquired by freeze-drying the activated sludge in which the production of the extracellular polymer is increased after the production increase step S21. In the freeze-drying step S30 of this embodiment, extra moisture in the activated sludge after the high-load step S210 has been removed using a centrifuge, and the activated sludge is subject to a freeze-drying process at 20 °C or less.

According to the process S1 for producing the freeze-dried sludge described above, in the high-load step S210 of the production increase step S21, a high load is applied to the cells in the activated sludge so that an amount of the extracellular polymer in the activated sludge can be increased. The extracellular polymer produced by the cells contains a component with a hydrogen bonding group such as a saccharide, a protein, a nucleic acid, and a lipid. For this reason, hydrophilic groups of cytoplasmic bilayer surfaces in the cells are hydrogen-bonded to hydrogen bonding groups of the extracellular polymer, the cells of the microorganism are dehydrated, and thus the cells can be reinforced at a time of freeze-drying. Therefore, the amount of the extracellular polymer in the activated sludge is increased so that many cells in the activated sludge are reinforced, and thus destruction of cell walls of the cells due to moisture at a time of freeze-drying the activated sludge can be suppressed. In other words, the cells in the activated sludge can utilize an extracellular polymer, which has been retained previously, as a protective substance. Thus, the FD sludge can be acquired without adding a protective medicine from the outside.

A protective medicine such as skim milk and sodium glutamate added from the outside has a hydroxyl group. For this reason, the hydroxyl group is hydrogen-bonded to the hydrophilic groups of the cytoplasmic bilayer surfaces of the cells so that the cells of the microorganism are dehydrated, and thus the protective medicine reinforces the cells at the time of the freeze-drying the cells. However, such a protective medicine includes an organic component. For this reason, such a protective medicine is effective in protecting the cells at the time of freeze-drying the cells, but the organic component itself included in the protective medicine is a decomposition target of the activated sludge when the FD sludge is used for treating wastewater after the cells have been freeze-dried, and thus there is a concern that the organic component may inhibit wastewater treatment. However, the amount of previously retained extracellular polymer in the cells can be increased and can be utilized as a protective agent so that the FD sludge which does not inhibit the wastewater treatment can be acquired.

In the high-load step S210, as the production increase operation, a load higher than that of the acclimatization step S10 is applied to the cells in the activated sludge so that a concentration of the extracellular polymer can be rapidly increased.

This was made evident from the concentration of the extracellular polymer in an experiment in which a high load was actually applied to the activated sludge. To be specific, an experiment was performed to evaluate a polysaccharide serving as a representative indicator for the extracellular polymer. In this experiment, the activated sludge serving as a sample is hydrolyzed into monosaccharides instead of a polysaccharide which is not easily directly analyzed, and a sugar concentration in the activated sludge is measured as the concentration of the extracellular polymer by analyzing total sugars. Fig. 2 is a graph showing results of changes in sugar concentration when a load is applied to the activated sludge.

In this experiment, as shown in Fig. 2, the same load as in a normal acclimatization operation is applied to the activated sludge at a point A serving as an initial state so that treatment performance is gradually improved. As a result, the sugar concentration also gradually increases up to a point B as the number of days of operation of an acclimatization operation increases. Here, an amount of organic components which is 1.2 or more times the amount added to the activated sludge in the acclimatization operation when compared with the limiting amount for the treatment performance added to the activated sludge at the point B. Thus, the sugar concentration of the activated sludge steeply increases from the point B to a point C. After that, when a load is not applied to the activated sludge at the point C, the sugar concentration quickly decreases from the point C to a point D. In other words, it was confirmed that a concentration of the extracellular polymer increased when a load was applied to the activated sludge. Particularly, it was confirmed that a concentration of the extracellular polymer rapidly increased in the activated sludge when a high load was applied to the activated sludge.

Therefore, in the high-load step S210, as the production increase operation, a load higher than that of the acclimatization step S10 is applied to the cells in the activated sludge so that the concentration of the extracellular polymer can be easily increased.

### «Second embodiment»

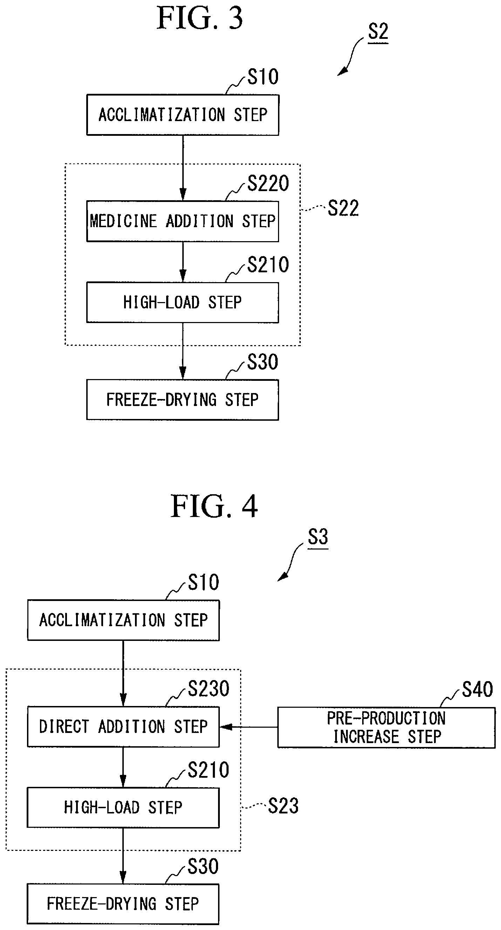

Next, a process S2 for producing freeze-dried sludge of a second embodiment will be described with reference to Fig. 3.

Constituent elements of the second embodiment the same as those of the first embodiment are denoted with the same reference numerals and detailed descriptions thereof will not be given. The process S2 for producing the freeze-dried sludge of the second embodiment is different from that of the first embodiment in that a medicine used for proliferating cells of a microorganism is added in a production increase step S22.

In the process S2 for producing the freeze-dried sludge of the second embodiment, as shown in Fig. 3, the production increase step S22 has a medicine addition step S220. In the medicine addition step S220, the medicine used for proliferating the cells is added.

In the medicine addition step S220, the medicine used for proliferating a type of cell in which a large amount of extracellular polymer is also secreted in the cells is added. The medicine addition step S220 is performed before a high-load step S210. Examples of a type of cell in which a large amount of extracellular polymer is secreted in this embodiment include, for example, Bacillus. As the medicine, for example, a medicine including a mineral component such as calcium, magnesium, and silicate is preferable.

According to the process S2 for producing the freeze-dried sludge of the second embodiment as described above, in the medicine addition step S220, the type of cell in which a large amount of extracellular polymer is secreted is proliferated by adding the medicine including the mineral component. As a result, the number of cells in the activated sludge increases, and thus the amount of extracellular polymer can be increased. In addition, the activated sludge may contain a lot of types of cell in which a large amount of extracellular polymer is secreted. As a result, in the high-load step S210, production of the extracellular polymer can be efficiently increased. Thus, in the production increase step S22, the production of the extracellular polymer can be increased in a short period of time.

### «Third embodiment»

Next, a process S3 for producing freeze-dried sludge of a third embodiment will be described with reference to Fig. 4.

Constituent elements of the third embodiment the same as those of the first embodiment and the second embodiment are denoted with the same reference numerals and detailed descriptions thereof will be omitted. The process S3 for producing the freeze-dried sludge of the third embodiment is different from those of the first embodiment and the second embodiment in that an additive generated from activated sludge in which production of an extracellular polymer has been increased in advance is added.

As shown in Fig. 4, the process S3 for producing the freeze-dried sludge of the third embodiment includes a pre-production increase step S40. In the pre-production increase step S40, activated sludge for addition is subjected to a production increase operation in which production of an extracellular polymer by cells of a microorganism is increased. A production increase step S23 of the process S3 for producing the freeze-dried sludge of the third embodiment includes a direct addition step (an addition step) S230. In the direct addition step S230, an additive generated from the activated sludge for addition in which production of the extracellular polymer has been increased after the pre-production increase step S40 is added.

The pre-production increase step S40 is performed before the production increase step S23. In the pre-production increase step S40, the activated sludge for addition is subjected to the production increase operation in which the production of the extracellular polymer is increased. The pre-production increase step S40 in this embodiment is performed before an acclimatization step S10. In the pre-production increase step S40, a load higher than that of the acclimatization step S10 is rapidly applied to the cells of the activated sludge for addition as in a high-load step S210 of the production increase step S23.

The direct addition step S230 of the production increase step S23 in the third embodiment is performed before the high-load step S210.

The direct addition step S230 is performed after the pre-production increase step S40. In the direct addition step S230, the activated sludge for addition in which the production of the extracellular polymer is increased is directly added as the additive.

According to the process S3 for producing the freeze-dried sludge of the third embodiment as described above, the activated sludge for addition in which the production of the extracellular polymer has been already increased is used. As a result, the amount of extracellular polymer in the activated sludge can be increased from an initial stage of the production increase step S23. In addition, the number of cells in the activated sludge increases. As a result, the production of the extracellular polymer can be efficiently increased in the high-load step S210. Thus, a time taken for the production of the extracellular polymer to be increased can be shortened in the production increase step S23. Therefore, the production of the extracellular polymer can be more efficiently increased.

In the direct addition step S230, the activated sludge for addition in which the production of the extracellular polymer has been increased is directly added as the additive. Thus, the amount of extracellular polymer in the activated sludge can be easily increased.

### «Fourth embodiment»

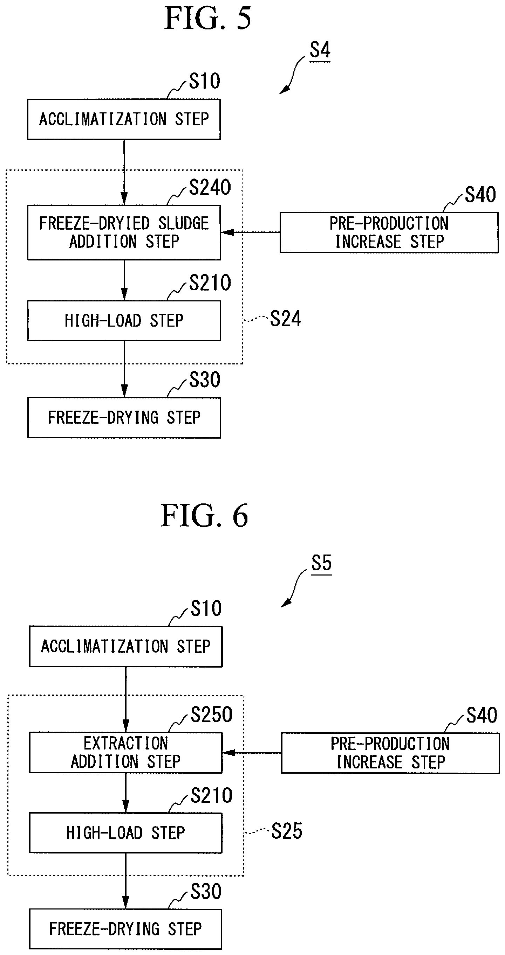

Next, a process S4 for producing freeze-dried sludge of a fourth embodiment will be described with reference to Fig. 5.

Constituent elements of the fourth embodiment the same as those of the first to third embodiments are denoted with the same reference numerals and detailed descriptions thereof will be omitted. The process S4 for producing the freeze-dried sludge of the fourth embodiment is different from that of the third embodiment in view of an addition step.

In the process S4 for producing the freeze-dried sludge of the fourth embodiment, as shown in Fig. 5, a production increase step S24 has a freeze-dried sludge addition step (an addition step) S240. In the freeze-dried sludge addition step S240, freeze-dried activated sludge for addition is added instead of the direct addition step S230.

In the freeze-dried sludge addition step S240, the activated sludge for addition in which production of an extracellular polymer after a pre-production increase step S40 has increased is freeze-dried and added as an additive.

According to the process S4 for producing the freeze-dried sludge of the fourth embodiment as described above, as in the third embodiment, the activated sludge for addition in which the production of the extracellular polymer has been already increased is used in the freeze-dried sludge addition step S240. As a result, the amount of extracellular polymer in the activated sludge can be increased from an initial stage of the production increase step S24. In addition, the number of cells of a microorganism in the activated sludge increase. As a result, the production of the extracellular polymer can be efficiently increased in a high-load step S210. Thus, a time taken for the production of the extracellular polymer to be increased can be shortened in the production increase step S24. Therefore, the production of the extracellular polymer can be more efficiently increased.

In the freeze-dried sludge addition step S240, the activated sludge for addition in which the production of the extracellular polymer is increased is freeze-dried and added as the additive. As a result, the activated sludge in which the amount of extracellular polymer has been increased can be reliably added even if a certain amount of time is taken until the production increase step S24 is performed after the pre-production increase step S40.

### «Fifth embodiment»

Next, a process S5 for producing freeze-dried sludge of a fifth embodiment will be described with reference to Fig. 6.

Constituent elements of the fifth embodiment the same as those of the first to fourth embodiments are denoted with the same reference numerals and detailed descriptions thereof will be omitted. The process S5 for producing the freeze-dried sludge of the fifth embodiment is different from those of the third embodiment and the fourth embodiment in view of an addition step.

In the process S5 for producing the freeze-dried sludge of the fifth embodiment, as shown in Fig. 6, a production increase step S25 has an extraction addition step (an addition step) S250. In the extraction addition step S250, an extracellular polymer extracted from activated sludge for addition is added instead of the direct addition step S230 or the freeze-dried sludge addition step S240.

In the extraction addition step S250, only the extracellular polymer extracted from the activated sludge for addition in which production of the extracellular polymer after a pre-production increase step S40 has been increased is added as an additive.

According to the process S5 for producing the freeze-dried sludge of the fifth embodiment as described above, as in the third embodiment and the fourth embodiment, in the extraction addition step S250, the activated sludge for addition in which the production of the extracellular polymer has been already increased is used. As a result, the amount of extracellular polymer in activated sludge can be increased from an initial stage of the production increase step S25. In addition, the number of cells in the activated sludge increase. As a result, the production of the extracellular polymer can be efficiently increased in a high-load step S210. Thus, a time for the production of the extracellular polymer to be increased can be shortened in the production increase step S25. Therefore, the production of the extracellular polymer can be more efficiently increased.

In the extraction addition step S250, only the extracellular polymer is added as the additive so that the amount of extracellular polymer in the activated sludge can be more efficiently increased.

Although the embodiments of the present invention have been described in detail above with reference to the drawings, constitutions and a combination thereof in the embodiments are merely examples. Addition, omission, substitution, and other modification of a constitution is possible without departing from the scope of the present invention. The present invention is not limited to the embodiments, and is only limited by the scope of the appended claims.

Note that the production increase steps S21, S22, S23, S24, and S25 are not limited to only implementation of the high-load step S210, and as long as production of an extracellular polymer can be increased any can be implemented by these. For example, the production increase step S21, S22, S23, S24, and S25 may independently perform the medicine addition step S220, the direct addition step S230, the freeze-dried sludge addition step S240, or the extraction addition step S250 without performing the high-load step S210.

The pre-production increase step S40 is not limited to implementation of the high-load step S210 and can be adopted for the present invention as long as it can increase production of an extracellular polymer in activated sludge for addition as in the production increase step S21, S22, S23, S24, or S25. For example, the pre-production increase step S40 may perform an acclimatization operation as in the acclimatization step S10 or may perform the medicine addition step S220.

The addition step is not limited to implementation of only any of the direct addition step S230, the freeze-dried sludge addition step S240, and the extraction addition step S250 and as long as an additive generated using activated sludge for addition in which production of an extracellular polymer has been increased can be added, any step can be adopted for the present invention. For example, the addition step may include implementation of all or a combination of two of the direct addition step S230, the freeze-dried sludge addition step S240, and the extraction addition step S250.

### [Industrial Applicability]

According to the process for producing the freeze-dried sludge described above, freeze-dried activated sludge can be obtained by increasing production of an extracellular polymer without adding a protective medicine from the outside.

### [Reference Signs List]

S1, S2, S3, S4, S5 Process for producing freeze-dried sludge
S10 Acclimatization step
S21, S22, S23, S24, S25 Production increase step
S210 High-load step
S30 Freeze-drying step
S220 Medicine addition step
S40 Pre-production increase step
S230 Direct addition step (addition step)
S240 Freeze-dried sludge addition step (addition step)
S250 Extraction addition step (addition step)

## Claims

1. A process for producing freeze-dried sludge comprising:
an acclimatization step of imposing a load on cells of a microorganism in activated sludge to acclimatize the cells;
a production increase step of subjecting the activated sludge to a production increase operation in which production of an extracellular polymer by the cells is increased after the acclimatization step; and
a freeze-drying step of freeze-drying the activated sludge after the production increase step.

2. The process for producing the freeze-dried sludge according to claim 1, wherein the production increase step comprises a high-load step of imposing a higher load than that of the acclimatization step on the activated sludge as the production increase operation.

3. The process for producing the freeze-dried sludge according to claim 1 or 2, wherein the production increase step has a medicine addition step of adding a medicine in which the cells are proliferated.

4. The process for producing the freeze-dried sludge according to any one of claims 1 to 3, further comprising:
a pre-production increase step of subjecting activated sludge for addition to a production increase operation in which the production of the extracellular polymer by the cells is increased,
wherein the production increase step has an addition step of adding an additive generated from the activated sludge for addition after the pre-production increase step to the activated sludge.

5. The process for producing the freeze-dried sludge according to claim 4, wherein, in the addition step, at least the activated sludge for addition is directly added as the additive.

6. The process for producing the freeze-dried sludge according to claim 4 or 5, wherein, in the addition step, at least the freeze-dried activated sludge for addition is added as the additive.

7. The process for producing the freeze-dried sludge according to any one of claims 4 to 6, wherein, in the addition step, at least the extracellular polymer extracted from the activated sludge for addition is added as the additive.
